# EUROPEAN PATENT SPECIFICATION

(11) **EP 0 412 700 B1**
(45) Date of publication and mention of the grant of the patent: **11.03.1998**
(21) Application number: 90308345.9
(22) Date of filing: 30.07.1990
(51) Int. Cl.: C12P 21/08, G01N 33/577

(54) **Method for isolating fetal cytotrophoblast cells**
Verfahren zur Isolierung von fetalen Zytotrophoblastzellen
Méthode pour isoler des cellules de cytotrophoblaste foetal

(30) Priority: 03.08.1989 US 389224
(43) Date of publication of application: 13.02.1991
(73) Proprietor: THE REGENTS OF THE UNIVERSITY OF CALIFORNIA, Oakland, California 94612-3550 (US)
(72) Inventor: Fisher, Susan, San Francisco, CA 94122 (US); Damsky, Caroline H., Belmont, CA 92004 (US); Librach, Clifford, Foster City, CA 94409 (US)
(74) Representative: Goldin, Douglas Michael

(56) References cited:
- WO-A-90/06509
- CHEMICAL ABSTRACTS, vol. 103, no. 23, 09 December 1985, page 346, abstract no. 192486c, Columbus, Ohio, US; B.H. BUTTERWORTH et al.: "Human cytotorophoblast studied by monoclonal antibodies using single and double biotin-avidin- peroxidase immunocytochemistry"
- Histochemical Journal 19, 288-296 (1987)
- Lancet 336, 197-200 (1990)
- Am J Obstet Gynecol 170, 1297-1300 (1994)
- Ann NY Acad Sci 731, 181-187 (1994)
- Clin Biochem Revs 15, 21-24 (1994)
- Ann NY Acad Sci 731, 162-169 (1994)
- Hawes et al., Proceedings 7th International Conference on Early Prenatal Diagnosis of Genetic Diseases, Jerusalem, Israel 22-27 May 1994, Monduzzi Editore, Bologna (Italy), pp. 219-222 (1994)
- J Clin Invest 89, 210-222 (1992)

## Description

The present invention is related to the detection of genetic disorders via prenatal diagnosis during early pregnancy, using relatively noninvasive means.

A variety of genetic disorders are detectable while the fetus is in utero. Some of these disorders have the potential of being extremely debilitating, in which case it may be desirable to allow the parents the decision to terminate the pregnancy. However, the most frequently used prenatal diagnostic techniques, amniocentesis and chorionic villus sampling (CVS), have significant drawbacks. These techniques require interruption of the fetal or placental membranes. Moreover, amniocentesis, although it presents little risk of associated complications, cannot be performed until midway through the second trimester. If termination is indicated, this timing in combination with the time required for the genetic analyses, results in the mother facing the termination late in the second trimester of pregnancy, which greatly enhances the health risk to the mother. CVS, which can be performed after the sixth week of pregnancy, carries with it a substantial incidence of abortion, which is reportedly as high as 12%. In addition, other associated risks, such as difficulty in obtaining chorionic villi suitable for karyotyping, have prevented widespread use of this technique.

Methods of prenatal diagnosis which utilize fetal cells obtained from maternal blood would eliminate the problems inherent in the above-described diagnostic techniques. However, significant advances towards this goal have been hampered by a major obstacle, i.e., the appearance of fetal cells in maternal blood is usually a relatively rare event. In fact, their detection usually requires highly specialized methods, such as fluorescence-activated cell sorting.

### DESCRIPTION OF THE RELATED ART

Attempts have been made to isolate fetal cells from maternal blood by fluorescence activated cell sorting. Herzenberg et al. (1979). These separations were based on the detection of labeled antibody probes which would bind HLA A2 negative cells in maternal blood. Male fetal cells separated in this manner were further identified by quinacrine staining of Y-chromatin. The isolated cells appeared to be lymphoid in origin. The definitiveness of this assay, however, is questionable since the difference in the quinacrine staining between the fetal male cells and the maternal female cells was small. In addition, the isolated fetal cells generally could not be stimulated to mitosis, an event which is essential for karyotyping and banding analysis.

Others have also used the fluorescence-activated cell sorter to detect fetal Y-chromatin-positive cells in maternal blood. Although the origin of these cells is unclear, their morphology resembles that of lymphocytes. Their unresponsiveness to phytohemagglutinin [Zillacus et al. (1975)], suggests that they may be either lymphocyte or erythrocyte precursors [Parks and Herzenberg (1982)]. The observed frequency of such cells ranged from less than 0.2% [Zillacus et al. (1975)] to 4% [Siebers et al. (1975)].

An attempt has been made, also, to recover fetal trophoblasts from maternal blood by flow cytometry. Covone et al. (1984). The monoclonal antibody used in these studies, H315, was produced using microvilli isolated from the syncytiotrophoblastic brush border of term placentas. It reportedly identifies a glycoprotein that is expressed on the surface of the human syncytiotrophoblast as well as other trophoblast cell populations, and that is absent from peripheral blood cells. A subpopulation of cells, fractionated using a ficoll-triosil gradient, was used for the sorting experiments. The cells or cell materials described were: 1) polynucleated syncytial cells found in 80% of the maternal cells at a frequency of 1-4/1000; 2) diploid cells, found in 50% of the samples with a frequency as high as 8/1000; and anucleate cell fragments, probably also derived from the syncytium, found in 33% of the samples at a frequency of <3/1000. The morphology of the fetal cells was not shown, and there was no attempt to further characterize the antibody-positive cells. In addition, the antibody used also recognized aggregated or polynucleated cells from non-pregnant women.

A method for isolating placental cell populations from suspensions of human placenta has been described by Kawata et al. (1984). The method uses coordinate two-color and light-scatter fluorescence-activated cell sorter (FACS) analysis and sorting. Five different cell populations were isolated on the basis of size and quantitative differences in the coordinate expression of cell surface antigens detected by monoclonal antibodies against an HLA-A,B,C monomorphic determinant (MB40.5) and against human trophoblasts (anti-Trop-1, and anti-Trop-2). Both anti-Trop-1 and anti-Trop-2 reportedly bound to a class of cells which were intermediate/large in size, and had some characteristics of fetal cells, but which did not exhibit HLA-A,B,C antigens.

Loke and Butterworth (1985) describe two monoclonal antibodies, 18B/A5 and 18A/C4, which are reactive with first trimester cytotrophoblasts. However, these monoclonal antibodies are also reactive with other fetal epithelial tissues. In addition, 18B/A5 also reacts with syncytiotrophoblasts.

Published International Application WO 90/06509, having a priority date of 6th December 1988 and published on 14th June 1990 after the priority date of the present application, describes prenatal diagnosis by isolation of villous Syncytiotrophoblast cells and non-villous cytotrophoblast cells from maternal blood samples using monoclonal antibodies reactive with both these cell types. Mueller et al (1987) Histochemical Journal 19, 288-296 also discloses such an antibody.

As hereinbefore indicated, isolation of fetal cells from maternal blood is desired to eliminate the problems inherent in conventional techniques currently commonly used to obtain fetal tissue for prenatal genetic analyses. However, significant advances toward this goal have been hampered by two major obstacles. First, the appearance of fetal cells in maternal blood is usually a relatively rare event, requiring highly specialized methods, such as fluorescence-activated cell sorting, for detection. Second, a wide spectrum of cell types, including those from the placenta and fetal blood may enter the maternal circulation. The current invention provides a solution to these problems by providing methods and compositions which are useful for the isolation of fetal cytotrophoblasts from maternal blood during the first trimester of pregnancy, thus permitting prenatal genetic analyses during early pregnancy, utilizing relatively noninvasive techniques for obtaining the foetal samples.

Accordingly, one object of the invention is a method for preparing a hybridoma which produces monoclonal antibodies which are:
(i) reactive specifically with the cytotrophoblast layer in sections of first trimester human placental chorionic villi;
(ii) reactive *in vitro* with the cytotrophoblast cells of the invasive type; and
(iii) not reactive with other cells in the peripheral blood;
the method comprising:
a. providing a purified preparation of first trimester cytotrophoblast cells from chorionic villi from which the layer of syncytiotrophoblastic cells has been removed;
b. immunizing an individual with the preparation of step a;
c. immortalizing antibody-producing cells of the individual immunized in step b; and
d. isolating clones of antibody-producing immortal cells which produce antibodies that have properties (i) to (iii).

The monoclonal antibodies produced by this method may also be:
(iv) reactive with purified preparations of dissociated cytotrophoblast cells of both the precursor and invasive types.

Yet another object of the invention is a monoclonal antibody, obtainable from the hybridoma as defined above, which is reactive with first trimester invasive cytotrophoblast cells and not reactive with peripheral blood cells.

Still another object of the invention is a method for isolating first trimester foetal cytotrophoblast cells from maternal blood comprising:
a. providing a sample of maternal blood;
b. interacting the maternal blood sample with monoclonal antibodies obtainable from the hybridoma as defined above, which are reactive with first trimester invasive cytotrophoblast cells and not reactive with peripheral blood cells, under conditions which allow an immunologically specific reaction; and
c. isolating the antibody-cytotrophoblast complexes from the remainder of the maternal blood components.

Still another object of the invention is a method for detecting first trimester fetal cytotrophoblasts in maternal blood comprising:
a. providing a sample of maternal blood;
b. interacting the maternal blood sample with monoclonal antibodies obtainable from the hybridoma as defined above, which are reactive with first trimester invasive cytotrophoblast cells and not reactive with peripheral blood cells, under conditions which allow an immunologically specific reaction; and
c. detecting the formation of antibody-cell complexes.

Still another object of the invention is the hybridoma ATCC No. 10097 and progeny thereof.

Further preferred embodiments of the invention will be apparent from the following description and claims.

### Brief Description of the Drawings

Fig. 1 is a diagram showing the anatomy of the maternal-foetal interface, including the sources of foetal cells in maternal blood. Panel (a), floating villus; panel (b), anchoring villus, and panel (c), broken villus.

Fig. 2 is a diagram showing two populations of cytotrophoblast cells found within the placenta. Panel (a) shows the stem cells that fuse to form the syncytium. Panel (b) shows the subpopulation of cytotrophoblast cells that invade the uterus and maternal vessels, eventually replacing the endothelial lining.

### Modes for Carrying out the Invention

The invention highlights methods and compositions useful for the isolation from maternal blood of foetal cells suitable for prenatal diagnosis. An understanding of the significance of the various embodiments of the invention, and of the foetal cytotrophoblast as a target cell for isolation or detection in maternal blood, is aided by an assessment of the possible origins of fetal placental cells which could enter the maternal circulation, and thus be available for detection.

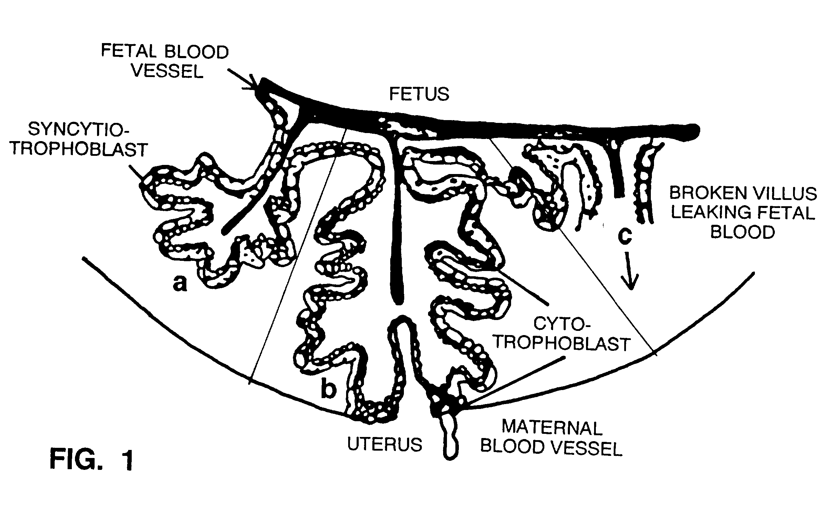

The anatomy of the maternal-fetal interface is shown in Fig. 1. In the interface, two types of fetal cells are in contact with maternal blood, syncytiotrophoblast cells and cytotrophoblast cells. The chorionic villi are covered by syncytiotrophoblast cells; these syncytial or multi-nucleate cells are probably the most numerous fetal cell type lying directly at the maternal-fetal interface, and are indicated by an "a" in Fig. 1. The microvillous brush border which covers these cells lacks class I and class II histocompatibility antigens. There is little probability that intact synctiotrophoblastic cells, or the chorionic villi which they cover, would circulate for prolonged periods in maternal blood. These multinucleate cells are extremely large (>100 microns) because of their syncytial nature. Thus, during circulation they would probably be trapped in the maternal capillary beds in, for example, the lungs and the liver. Histological examination has revealed multinucleate syncytiotrophoblastic cells lodged in the lungs of pregnant women [Atwood and Park (1961)], and immediately following birth, in blood drawn from vessels which drain the uterus [Douglas et al. (1959)].

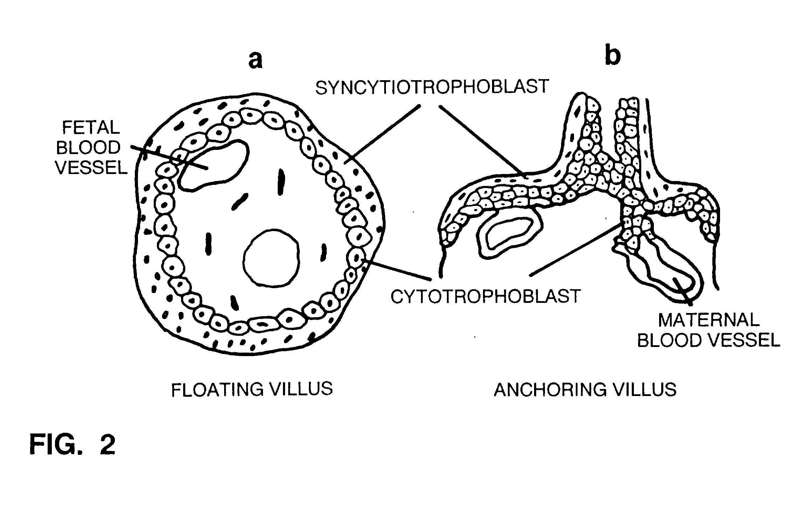

Another cell type found at the maternal-fetal interface is the cuboidal cytotrophoblast cell, which contains 1 nucleus per cell, and which is approximately 20 microns in size. The relative locations of these cells are shown in Fig. 1, and Fig. 2. Types of cytotrophoblast cells are divisible into two different populations, as evidenced by morphological criteria, and by their different functions [Enders (1968)]. The relative locations of the two populations of cytotrophoblasts are shown in Fig. 2. One population of cytotrophoblast cells lies beneath the syncytium. These cells are precursors to the syncytiotrophoblastic cells, which result from the fusion of the cytotrophoblasts (Fig. 2a). The subset of precursors lies buried within the chorionic villus and is probably only rarely exposed to maternal blood. These precursor cells are most numerous during the first trimester, rapidly diminish in number during the second trimester, and are almost totally absent by term. The chorionic villi which contain this precursor subset of cytotrophoblasts are called "floating villi", since they are not attached to the uterus, but rather float in maternal blood. Placentas obtained by vacuum aspiration during the first trimester, and term placentas, contain primarily floating villi.

A second population of cytotrophoblast cells are "invasive" cytotrophoblasts. Masses of these cells, termed "cell columns" burrow through the syncytiotrophoblast cells which cover the chorionic villi and invade the uterus. Eventually the cell columns erode the uterine vessels, and cytotrophoblast cells replace the maternal endothelial lining [Pijnenborg (1981)]. (See panel b, Fig. 1, and panel b, Fig. 2.) The anchoring chorionic villi formed by this process attach the placenta to the uterus, since the cell columns are continuous with the cytotrophoblast stem cells found in the chorionic villi. These invasive cytotrophoblast cells are attached to maternal blood vessels, and thus are in contact with maternal blood. (See panel b of Fig. 2). Moreover, they are single cells of a relatively small size, which would not be filtered out during circulation through maternal capillary beds.

Results have been obtained which are consistent with the suggestion that certain of the events which occur during the early phases of human placentation in vivo can be duplicated in vitro. Initial experiments demonstrated that first trimester cytotrophoblast cells migrating from placental villi invaded extracellular matrices produced by bovine corneal endothelial cells (BCE-ECM) and by the PF-HR9 teratocarcinoma cell line [Fisher et al. (1985)]. Subsequently we ascertained that purified preparations of cytotrophoblastic cells also invaded this matrix (Fisher et al. (1989, a and b). In both cases, only a subpopulation of the population of cells in the villi or purified cells degraded the matrices on which they were cultured. This suggests that the cytotrophoblast cells isolated from first trimester human placentas, consisting primarily of the stem cells found in floating villi, are a mixture of those which can become invasive and those which fuse to form the syncytium. In addition, the results showed that cytotrophoblastic cells cultured in vitro retain the timetable of invasive behavior which they exhibit in vivo, where invasion peaks during the twelfth week of pregnancy. Second trimester villi and cytotrophoblast cells adhere to the matrices, but fail to invade.

Recently completed experiments (manuscript in preparation) have used another experimental system, devised for studying tumor cell invasion (Albini et al., 1987; Erkell and Schirrmacher, 1988), to investigate trophoblast invasion. Polycarbonate filters with 3, 5 and 8 um pores were evenly coated with Matrigel, a commercially available basement membrane-like material. First trimester and term cytotrophoblast cells were cultured on these matrix-coated filters. Several interesting findings have emerged from these experiments. Notably, the cytotrophoblast cells behave very differently when they are plated on different types of extracellular matrices. First trimester cytotrophoblasts plated as single cells on PF HR9 matrices form monolayers (Fisher et al., 1985, 1989), whereas equal numbers of first trimester cytotrophoblast cells plated on Matrigel rapidly form aggregates. In addition, many of these structures appear to be connected by elongated processes that contact adjacent aggregates. The structure of these aggregates resembles more closely that of a villus than does a monolayer of cytotrophoblast cells. In contrast, identical numbers of term cells plated on Matrigel fail to aggregate.

We have used this system to examine the invasive properties of the placental cells. After 18 h, sections were cut from cultures of first and third trimester cytotrophoblasts. The cells located at the base of first trimester structures appeared to have invaded the underlying ECM with the net effect that the entire structure seemed to sink into the matrix. That invasion had occurred was confirmed by the observation that many sections of the underlying Matrigel showed embedded cytotrophoblasts lying in hollowed out channels. In contrast, the term cytotrophoblast cells remained as single cells superficially attached to the Matrigel with no evidence of invasion.

We also used scanning electron microscopy to study Matrigel invasion by the cytotrophoblasts. We had previously observed that cytotrophoblasts found along the edges of holes in the PF HR9 matrix had extremely complex surfaces. Scanning electron microscopy showed that the cells interacting with the matrix do so by means of elaborate processes that radiate from the cell surface. The trophoblast processes appear to be associated with sites of matrix dissolution. We also examined the underside of the Matrigel-coated filters on which these cells were grown. First trimester cytotrophoblast cells were unable to penetrate filters with 3 µm pores, but sent numerous elaborate processes through these, as well as the 5 µ and 8 µm pores. The leading edge of the membrane was often ruffled, and again, numerous elaborate processes were visible. In general, these cellular projections appeared first, followed by the emergence of the entire cell through the pore. When first trimester cytotrophoblasts were plated on Matrigel coated filters with 8 pm pores, entire cells were able to penetrate. A low power SEM of the bottom of the filter showed that even after only 18 h in culture, numerous cells had migrated from the top surface through the Matrigel. Fibroblasts isolated from first trimester human placentas were also plated on Matrigel-coated filters. These cells formed a monolayer rather than aggregates. Examination of the underside of the filters showed that no cells penetrated the filters. Thus, first trimester human cytotrophoblasts both degrade (Fisher et al., 1985, 1989 a, b) and migrate through ECMs in vitro. In contrast, later gestation cytotrophoblast cells do neither. These results show that the cytotropnoblast cells maintain in vitro the developmentally regulated invasive behavior they exhibit in vivo. Furthermore, the invasive subpopulation of cytotrophoblast cells which emerges on the underside of the filter can be separated from the noninvasive cells which fail to penetrate the Matrigel.

Of consequence, also, for prenatal genetic analyses, is the finding that first trimester human cytotrophoblast cells can be maintained in culture. Cells which arise from the fetal circulation do not appear to have this attribute.

Based on the above, the methods and compositions of the invention pertain to the identification in, and isolation from, maternal blood of fetal cells which exhibit the characteristics of first trimester cytotrophoblasts, since these are the cells which are most likely to reach the maternal circulation, and to not be subsequently removed in the maternal capillary beds.

In the invention, a panel of monoclonal antibodies are generated which are specific for antigens expressed by first trimester human cytotrophoblasts. During gestation the placenta undergoes profound morphological alterations, which reflect changes in many biochemical and functional properties. These morphological and functional alterations suggest that antigens which are present on cytotrophoblast cells early in pregnancy may be missing or altered on cytotrophoblast cells from term placentas. For example, certain proteases and integrins (cell adhesion molecules) are unique to first trimester cells (Fisher et al., 1989 b). Thus, in the invention, hybridomas which produce the monoclonal antibodies specific for cytotrophoblast antigens are created by using as an immunogen, a purified preparation of first trimester cytotrophoblast cells. This preparation consists of invasive cells, and of those which fuse to form the syncytium. The first trimester cytotrophoblasts are used as the immunogen in order to increase the possibility of producing monoclonal antibodies which specifically detect antigens expressed by these cells.

The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., M. Schreier et al., (1980); Hammerling et al., (1981); Kennett et al., (1980); see also,U.S. Patent Nos. 4,341,761; 4,399,121; 4,427,783; 4,444,887; 4,466,917; 4,472,500; 4,491,632; and 4,493,890. Methods for producing the monoclonal antibodies utilizing the isolated cytotrophoblast cells as immunogen are disclosed infra.

The monoclonal antibodies are then screened to identify those which are useful for detecting a majority of first trimester cytotrophoblast cells, including those which are precursor to syncytiotrophoblasts, and those which are invasive. The screening consists of multiple steps requiring varying degrees, usually increasing, of stringency of specificity. The screening steps include the following.
1. Isolating antibodies which react specifically with the cytotrophoblast layer in sections of first trimester human placental chorionic villi.
2. Isolating antibodies which react with cytotrophoblast cells, and which do not react with peripheral blood components.
3. Isolating antibodies which react with purified preparations of dissociated cytotrophoblast cells, of both precursor and invasive type.
4. Determining whether antibodies which satisfy criteria 1-3 also react with first trimester human cytotrophoblast cells which are actively invading extracellular matrices in vitro.

The screening procedures are designed to rigorously test the specificity of the expression of the antigen(s) identified by the monoclonal antibodies produced against first trimester cytotrophoblast cells. The strategy reflects two important considerations. First, the initial procedures using sections of first trimester placentas and peripheral blood smears are rapid and relatively easy to perform. Thus, these procedures allow the screening of large numbers of antibodies. Second, the final two procedures (steps 3 and 4) are more difficult to perform, and thus are not used as a general screen for large numbers of antibodies. In the screening method, the numbers of antibodies to be screened by these procedures are already significantly reduced because of the first two screening steps.

The initial screening on sections identifies antibodies which react with cytotrophoblasts which are located beneath the syncytiotrophoblastic covering of first trimester chorionic villi. These cytotrophoblasts are precursor cells, which can either fuse to form the syncytium, or acquire invasive characteristics. Thus, these cells are expected to express antigens which are common to a majority of cytotrophoblast cells.

Another material which may be used in the initial screening step are sections of placental bed. Uterine tissue, and particularly blood vessels, contains invasive cytotrophoblast cells.

The second step in the screening process is to eliminate antibodies which react with maternal blood. If the monoclonal antibodies of the invention are to be successfully used to distinguish fetal cells from maternal blood cells, this step or its equivalent is particularly important. A substantial body of evidence indicates that lymphocytes and trophoblast cells share common antigens. Cross-reactivity of antisera between rodent trophoblasts and peripheral blood lymphocytes has been reported by Beer et al. (1972). A similar class of human antigens has been termed TLX (trophoblast-lymphocyte cross-reactive antigens) by McIntyre and Faulk (1979), and the antibody used by Herzenberg and coworkers may have identified antigens in this particular class [Herzenberg et al. (1979)].

The screening in the second step may be accomplished using any technique which monitors the binding or lack of binding of antibodies to peripheral blood cells. These techniques are known to those of skill in the art. Preferably, however, for the reasons cited above, the screening is performed using peripheral blood smears. The antibodies which fail to react with the component cells in control blood smears (from individuals who are not pregnant), may then be further screened, using a high speed cell sorter, to confirm that the antibodies lack immunoreactivity with blood cells.

The third step in the screening procedures determines whether monoclonal antibodies which specifically identify cytotrophoblast cells in chorionic villi, but which do not react with peripheral blood components, stain the majority of cells in a preparation of purified cytotrophoblast cells. A positive reaction with a majority of cells is indicative that the antibody identifies both the precursor cells which fuse to form the syncytium, and invasive cells.

The last screening step is to determine whether antibodies which satisfy the preceding criteria also react with cytotrophoblast cells which are actively degrading extracellular matrices in vitro. Invasion of the maternal tissues precedes access to the maternal circulation, and the possibility exists that the development of the invasive phenotype is concurrent with the destruction of some of the antigens which are detected in the other steps of the screening procedure. Thus, this step ensures that the antibodies selected recognize antigens which are common to both precursor and invasive cytotrophoblasts. An in vitro system which is suitable for this screening is described in Fisher et al. (1985, 1989 a, b), and is further elucidated infra.

The monoclonal antibodies isolated by the above described screening procedure are used for the detection and/or isolation of cytotrophoblasts in samples of maternal blood. In this procedure, the antibodies are mixed with maternal blood, and the antibody-cell complexes are identified and/or separated from the remainder of the blood cells. The identification may be by labeling the antibody with an appropriate label. Appropriate labels, and the methods of attaching them to antibodies are known to those of skill in the art, and include fluorescent, chemiluminescent, radioactive, and dye molecules. Techniques which amplify the signals from the labeled antibodies are also known; for example, detection systems which utilize biotin and avidin, and enzyme-labeled and mediated immunoassays. A preferred system of detection and separation utilizes fluorescein to label the antibodies, followed by separation using a high speed fluorescence activated cell sorter.

It may be desirable to attach the anticytotrophoblast monoclonal antibody to a solid phase to accomplish the isolation and/or detection of the cytotrophoblasts. Methods of attaching monoclonal antibodies to solid phases are known in the art, and include those which involve hydrophobic, ionic, and chemical bonding of the monoclonal antibody to the solid phase. Solid phases which are useful are known in the art, and could include, for example, beads which may or may not be magnetic, plates, and strips of polymeric materials. In the event that the monoclonal antibodies are attached to magnetic beads, it may be possible to isolate the antibody-cell complexes utilizing a magnetic cell separator.

In addition, it may be desirable to enrich the concentration of cytotrophoblasts in the maternal blood sample prior to the specific reacting with the anticytotrophoblast monoclonal antibodies. This may be accomplished by positive or negative selection techniques. For example, for a positive selection technique, a prescreen cocktail may contain monoclonal antibodies which are cytotrophoblast specific, and/or monoclonal antibodies which bind to epitopes which are present on cytotrophoblasts and on other cell types, but not on blood cells. Examples of the latter include anticytokeratins. Our studies indicate that first trimester cytotrophoblasts contain cytokeratins, and anticytokeratins have been used to screen for cytotrophoblasts from maternal blood. Khong et al. (1986). In addition, cytokeratins are not expected to normally be a constituent of blood cells.

Other methods of enrichment could rely on negative selection techniques. For example, antibodies which do not bind cytotrophoblasts, but which bind other cells in the blood, may be used initially to remove those cell types from the sample. These antibodies may be attached to a solid phase to aid in the separation, as described above. Antibodies which bind to various types of cells in the blood are known in the art, and include, for example, HLE-1 which are specific for monocytes. The preparation from which other cell types have been removed, but which contain cytotrophoblasts, may then be treated with anticytotrophoblast monoclonal antibodies to isolate and/or detect the cytotrophoblasts.

Isolation and/or detection of antibody-cell complexes may be accomplished utilizing devices which allow image analysis; these devices are known by those of skill in the art, and include, for example, those which utilize light microscopy, optical instrumentation, fluorescence scanning, etc.; these devices may be computer assisted in the scanning. In addition, the devices may monitor the flow of suspensions suspected of containing the antibody-cell complexes.

The anticytotrophoblast monoclonal antibodies isolated by the procedures described herein are useful for a variety of techniques used in prenatal diagnosis. For example, cells identified by this procedure can be grown in culture and karyotyped, using known procedures for karyotyping. The cells may also be used for the identification of restriction length polymorphisms which are known to be associated with a variety of genetic diseases, for example, the thalassemias, sickle cell anemia, juvenile insulin-dependent diabetes mellitus, and Huntington's disease. Individual DNA sequences which are associated with genetic diseases may also be detected by techniques utilizing, for example, DNA amplification techniques, such as the polymerase chain reaction technique.

In addition, it has been shown that elevated numbers of circulating trophoblast cells may be associated with certain pathological conditions such as preeclampsia. Jaameri et al. (1965). This fatal complication increasing in frequency usually appears during the second trimester and is characterized by maternal hypertension, proteinuria, and fetal intrauterine growth retardation. It is feasible that, prior to the development of overt symptoms, maternal blood samples during the late phase of the first trimester may be monitored for increased levels of circulating cytotrophoblasts using the anticytotrophoblast monoclonal antibodies to determine whether this condition is developing.

Described below are examples of the present invention which are provided only for illustrative purposes, and not to limit its scope. In light of the present disclosure, numerous embodiments within the scope of the claims will be apparent to those of ordinary skill in the art. Although specific hybridomas are designated in the Examples, equivalents of these hybridomas are readily recognized by those of skill in the art. Equivalent hybridomas include those which produce antibodies which react immunologically with epitopes with which the antibodies produced by the designated hybridomas are immunologically reactive, although the avidity of binding may differ. Techniques for determining whether antibodies are immunologically reactive with the same epitopes are known in the art, and include, for example, competitive binding studies.

### EXAMPLES

### Isolation of First Trimester Cytotrophoblast Cells

Cytotrophoblast cells are isolated from first trimester human placentas. The placentas are obtained immediately after vacuum aspiration, and washed in several volumes of phosphate buffered saline (PBS) at 10°C to remove as much of the remaining maternal blood as possible. Any portions of the placenta from which clotted blood could not be removed are discarded. The fetal tissue are dissected free of adherent decidua and rinsed in several volumes of PBS until the white chorionic villi float freely in the buffer. Large villus pieces are removed from the placentas and transferred to DME H-21 containing 10% fetal calf serum (FCS) and 50 micrograms/ml gentamicin. After centrifugation (180 x g, 5 min) the medium is aspirated and the wet weight of tissue is determined. The villi are washed in two more changes of medium.

In order to isolate cytotrophoblast cells, the washed villus pellet is resuspended (5:1, v/w/w) in dissociation solution 1 [PBS containing 500 U/ml collagenase (Sigma, Type IV), 200 U/ml hyaluronidase (Sigma, Type 1-S), 0.2 mg/ml DNase (Sigma, type IV) and 1 mg/ml bovine serum albumin (BSA)]. The suspension is gently shaken in a 37°C water bath. The incubation time necessary to remove the layer of syncytiotrophoblast cells covering the chorionic villi is assessed histologically for every lot of collagenase, and for placentas of different ages. In general, first trimester tissue is incubated for 20 min. The first dissociation solution, containing syncytium, is removed and the villi are resuspended in dissociation solution II [PBS containing 0.25% trypsin (Sigma, Type XIII), 2 mM EDTA and 0.2 mg/ml DNase. After incubation in a shaking water bath at 37°C for 10 min, the mixture of villus cores and dissociated cells is diluted with an equal volume of medium containing 10% FCS. The villi and medium are poured through gauze to remove large tissue fragments, and the supernatant is centrifuged as described above. The incubation time necessary to remove the cytotrophoblast layer is assessed histologically. Generally, a single cycle removes the cytotrophoblast cell layer from the chorionic villi.

The resulting cell pellets are resuspended in 4 ml of medium containing 10% FCS and layered over a preformed Percoll gradient made up in Hanks' balanced salt solution according to the method of Kliman et al. (1986). The gradient is formed from 70% to 15% Percoll (v/v) in 5% steps of 3 ml each by diluting 90% Percoll (9 parts Percoll:1 part 10X Hanks' balanced salt solution) with calcium- and magnesium-free (CMF) Hanks' balanced salt solution and layering in a 50 ml-conical polystyrene centrifuge tube. The gradient is centrifuged (1000 x g) for 25 min at room temperature, after which a broad band in the middle of the tube containing the cytotrophoblast cells is removed and diluted in a 10X volume of medium. The cells are isolated by centrifugation, washed two more times and resuspended in MEM D-valine medium [Gilbert and Migeon (1975)] containing 20% dialyzed FCS, 1% glutamine and 50 micrograms/ml gentamicin. Cytotrophoblast cells are counted in a hemocytometer and adjusted to a final concentration of 5 x 10⁵/ml. The yield of cytotrophoblast cells is approximately 10⁵ to 10⁶ cells per first trimester placenta.

The isolated preparations of cytotrophoblasts contain <1% fibroblasts. To prevent growth of the fibroblasts in culture preparations which will be maintained for periods greater than 1 week, a D-valine containing medium is used.

### Production of Anticytotrophoblast Monoclonal Antibodies

Monoclonal antibodies are raised using as immunogens intact cytotrophoblasts, cytotrophoblast plasma membranes, or detergent extracts of cytotrophoblasts, or isolated molecules such as cytokeratins, Fc receptor processes, and cell adhesion molecules.

Cytotrophoblast plasma membranes are isolated according to Brunette and Till (1971). Detergent extracts are prepared by the procedure of Knudsen (1985). In the latter procedure, the cells are extracted with Triton™ X-114 in the cold, and brought to a "cloud point" at 30°C. After centrifugation of the extract, the separated phases are collected.

Immunization of mice with the immunogen is carried out as follows. The immunogen is injected with complete (injection 1) or incomplete (injections 2 and 3) Freund's adjuvant intraperitoneally at 3 wk intervals into BALB/C mice. The final series of injections are three daily tail vein injections of immunogen without adjuvant.

Rats are also suitable for immunization to produce monoclonal antibodies. Cells or components are injected intraperitoneally with complete Freund's adjuvant. Two subsequent injections are given intrasplenically without adjuvant.

The third day after immunization, the spleens are removed from the immunized mice or rats, and the spleen cells are fused with SP2/0 mouse myeloma cells, essentially according to the procedure of Kennett (1980). The fused cells are plated at an expected density of 1 successful fusion product per microtiter well in 50 microliters Kennett H-Y medium. After 24 hrs, cells in each well are fed with 50 microliters H-Y plus 2X aminopterin to selectively kill unfused SP2/0 cells.

In order to screen for hybridomas producing the desired antibodies, the immunogen used for the particular fusion is immobilized to a 96-well micro-ELISA plates in a pH 9.0 buffer, as described by Voller et al. of Dynatech Laboratory, Alexandria, Va. An extract of cells or tissue not expected to express the antigen(s) of interest (e.g., placental fibroblasts or peripheral blood cells) is used as the negative control. Hybridoma supernatants are added for 2 hrs at 4°C. After washing, the amount of antibody bound is determined by adding rabbit antimouse IgG conjugated to beta-galactosidase for 2 hr, followed by p-nitrophenyl beta-d-galactopyranoside (BRL, Rockville, Md.). Positive wells develop a bright yellow color. Positive hybridomas (i.e., hybridomas producing antibody that reacts with the immobilized immunogen, but not with the negative control) are cloned by limited dilution and rescreened. Positive clones are further screened using frozen and plastic embedded sections of first trimester chorionic villi, as described below. Hybridomas resulting from this selection are J1D8, N1C6, J2F6, P1B5, S8G4, J1B5, 10D3.D9, 10108.C9, J2F6, and J2E8.

Efficient production of monoclonal antibodies is accomplished by injecting about 107 cells of the selected hybridoma(s) intraperitoneally into Pristane-primed Balb/C mice. Ascites fluid are harvested, the cells in the fluid pelleted, and used to serially inject additional mice.

In order to purify the antibodies, those present in the ascites fluid or in culture medium conditioned by antibody-producing cells are purified by protein A-affinity chromatography. If the antibodies are the wrong isotype and do not bind to protein A, they are purified by affinity chromatography using immobilized antimouse immunoglobulin. Alternatively, all isotypes of mouse Ig may be purified using HPLC on a hydroxyapatite column (BioRad).

### Screening Monoclonal Anticytotrophoblast Antibodies on Sections of First Trimester Human Chorionic Villi

In order to determine whether hybridomas selected by the procedure described above produce antibodies which specifically react with cytotrophoblastic cells, the antibodies are screened using sections of chorionic villi from first trimester human placentas. A rapid procedure for determining antibody localization utilizes frozen tissue sections.

Frozen sections are prepared from first trimester human chorionic villi obtained immediately after vacuum aspiration. The tissues are fixed for 30 min with 3% paraformaldehyde in C PBS, pH 7.2. Samples are rinsed in CMF PBS containing 5% sucrose, and washed in CMF containing 0.1% glycine to quench unreacted aldehyde groups. After rinsing in CMF PBS containing 5, 10 and 15% sucrose, the tissues are embedded in OCT (Miles Scientific, Naperville, IL) and frozen in liquid nitrogen. Sections (5 microns) are cut using a Slee HR cryostat and collected on 22-mm² coverslips.

The antibodies are, alternatively or in addition, screened by staining semi-thin sections of material embedded in glycomethylacrylate (GMA, 38-41), or LR White according to Newman et al. (1983). Prior to staining, the coverslips are exposed for 10 min. to PBS containing 0.2% BSA to block nonspecific binding by antibodies.

The method for GMA embedding is as follows. The tissue is fixed using freshly prepared 3% paraformaldehyde in 0.1M phosphate buffer, pH 7.4, for 2 hr. After dehydration in graded acetone, the tissue is embedded in JB-4 (GMA as marketed by Polysciences, Inc.) by reacting the tissue as follows: 1) 50% acetone, twice for 10 minutes each; 2) 95% acetone, twice for 10 minutes each; 3) 100% acetone, twice for 10 minutes each; 4) 50% acetone:50% JB-4 monomer (Solution A), once for 10 minutes; 5) JB-4 monomer, 1 x 10 minutes; and 6) JB-4 monomer, overnight. The tissue is then placed in molds containing an embedding medium of JB-4 solution A (20 ml) benzol peroxide (0.09 g) and JB-4 solution B (0.5 ml, Polysciences, Inc.). The polymerization is done under vacuum (15-20 mm Hg) at 4°C for 12 hrs. After warming to room temperature, sections, 2 microns thick, are cut with a glass knife using a Sorvall JB-4 microtome. The sections are transferred via water to coverslips and air dried at room temperature. Prior to staining, coverslips with sections are washed in PBS and exposed to 3% normal goat serum (NGS) in PBS for 30 min at room temperature in a humidified chamber.

Immunological reactivity of the monoclonal antibodies is monitored. The preparations of antibodies to be tested are diluted in PBS with NGS and incubated with tissue sections for 30 to 60 min. The slides are washed with PBS-NGS and exposed for 30 min to either fluorescein or rhodamine-conjugated second antibodies with specificities appropriate to the species and immunoglobulin class of the monoclonal antibodies. The slides are examined using a fluorescent Zeiss microscope. Exposure to nonimmune serum, absence of first antibody, and use of an inappropriate second antibody serve as controls.

The second antibody may be peroxidase-conjugated. In that event, endogenous peroxidase activity is blocked by exposing sections to 0.3% hydrogen peroxide in methanol for 30 min, followed by a wash with PBS. The sections are incubated with peroxidase-conjugated second antibody (1:20-1:100) for 24 hr at 4°C, followed by a wash with PBS. The reaction is developed with diaminobenzidine (12.5 mg DAB in 100 ml PBS containing 5 microliters 30% hydrogen peroxide) for 10 min.

### Screening for Lack of Cross-Reactivity with Peripheral Blood Components

Initially, the anticytotrophoblast monoclonal antibodies are screened on cells fractionated from whole blood. Total white cells are isolated from peripheral blood by differential centrifugation in a Wintrobe tube. A drop of the buffy coat containing the white cells is smeared on a slide and lightly fixed using ice cold methanol acetone (3:1 v/v), before exposure to the antibodies. The antibody exposure is as described above.

Preparations of antibody which do not react with the white cells on slides are further screened for reactivity with peripheral blood components using a high speed cell sorter. In this case, the antibodies are fluorescein-labeled.

### Screening on Dissociated Cytotrophoblast Cells

Cytotrophoblast cells are isolated from first trimester human placentas using the procedure described above. Cell pellets are frozen in liquid nitrogen-cooled isopentane, and frozen cross-sections cut as described above. The frozen cross-sections are reacted with the monoclonal antibodies, and immunological reactivity is determined as described above.

Antibodies which react positively with the frozen sections are then screened for reactivity with the isolated cytotrophoblasts embedded in GMA. The embedding and reactivity monitoring are performed as described above, except that cell pellets containing the isolated cytotrophoblasts are substituted for the human chorionic villi.

### Determining Reactivity of Anticytotrophoblast Monoclonal Antibodies Against Invasive Cytotrophoblasts

The invasive cytotrophoblast cells used for this screening are grown on isolated extracellular matrices (ECM). The presence of invasive cells is evidenced by matrix clearing. Three different matrices may be used, which have different characteristics. The composition of the PF HR9 teratocarcinoma matrix resembles that of a natural basement membrane; major constituents include collagen type IV, laminin (both considered diagnostic of a basement membrane, entactin, and a heparan sulfate proteoglycan. Kramer et al. (1984). However, in contrast to a natural basement membrane, the degree of molecular cross-linking is minimal. The bovine corneal endothelial (BCE) cells secrete a hybrid matrix. This matrix has both basement membrane and interstitial matrix components and is also cross-linked. Robinson and Gospodarowicz (1983). Cross-linking of collagen and of elastin results in the formation of a physically stable, impenetrable layer. Thus, enzymes which are not necessary for HR9 matrix degradation may be required for BCE matrix invasion.

The PF-HR9 cell line was obtained from Dr. Erkki Ruoslahti at the La Jolla Cancer Research Foundation. The cells are routinely cultured in DME (0.45% glucose, DMEM-H) supplemented with 10% FBS (FBS, Sterile Systems, Logan, UT) and 10-50 micrograms/ml gentamicin. All cells are passaged at regular intervals using either trypsin alone, or trypsin-EDTA.

The PF-HR9-ECM is produced by seeding cells onto plastic coverslips, or into plastic wells, at 5 x 10⁵ cells per well. The substratum is precoated with bovine plasma fibronectin (50 micrograms/ml in PBS, 30 min at 25°C), isolated as described in Kramer et al. (1982) and Kramer et al. (1983). The culture medium is replaced every second day with DMEM-H containing 10% FBS, 50 micrograms/ml ascorbic acid, and 10 micrograms/ml gentamicin.

After approximately 10 days the PF-HR9-ECM is isolated using a modification of the procedure described previously by Kramer (1984). The coverslips or wells are washed twice with hypotonic buffer (10 mM Tris HCl, 0.5 mg/ml BSA, 0.1 mM CaCl₂, pH 7.5) at 37°C, then incubated with the same buffer for 10 min at 37°C, during which time the cells become swollen. Lysis is accomplished by a brief extraction (2 min, 37°C) with 0.5% NP-40 nonionic detergent diluted with hypotonic buffer. The coverslips or plates are washed four times with distilled water, incubated for 15 min. at room temperature with 0.1M ammonium hydroxide, rinsed three times with PBS containing 10% FBS, and then incubated for 1 hr in DMEM-H plus 10% FBS to extract any residual detergent. In all cases, the PF-HR9-ECM are used immediately after isolation.

Cultures of BCE cells are established from steer eyes according to Gospodarowicz et al. (1983). Stock cultures are maintained on gelatin-coated tissue culture dishes and grown in Dulbecco's modified Eagle's medium (0.1% glucose, DMEM-L) supplemented with 15% FBS, 2 mM glutamine and 50 micrograms/ml gentamicin. Fibroblast growth factor (FGF) isolated according to Gospodarowicz et al. (1984), is added to a final concentration of 1 ng/ml every other day until the cultures reach confluence. Stock cultures are passaged weekly at a split ratio of 1:64. Surfaces coated with BCE-ECM are produced by seeding the cells in the above medium supplemented with 3% dextran (40,000 MW). One week postconfluence, the BCE-ECM is denuded of cells by exposing the culture to 20 mM ammonium hydroxide for 5 min. The BCE-ECM is washed with Dulbecco's PBS(D-PBS) prior to storage at 4^{o}C in D-PBS plus antibiotics.

Isolated cytotrophoblast cells, prepared as described in the above Section, are plated on the ECM. Degradation of the matrix appears to begin only after the cells near confluence. In the case of the PF-HR9-ECM, which is less highly cross-linked, visible holes are usually formed within 48-72 hours, whereas comparable clearing in the BCE-ECM takes about twice the time. Invasion of the Matrigel-coated filters occurs with 18 h.

The screening for anticytotrophoblast monoclonal antibodies which are immunologically reactive with the invasive cytotrophoblasts is monitored utilizing the cells which have invaded the ECM on coverslips or through Matrigel-coated filters. The fixation of the material on the coverslips and filters and the immunological reactivity of the fixed material is as described above, for the thin sections of chorionic villi.

Screening is usually performed first on the cytotrophoblasts which invade the PF-HR9-ECM or Matrigel. If further screening is desired, the cells which invade the BCE-ECM are tested to confirm that, even after extensive invasion, cytotrophoblast-specific antigens can be detected.

Clones of hybridomas which appear to yield monoclonal antibodies with the desired specificity are 10D3.C9 and N1C6.

### Anticytotrophoblast Monoclonal Antibodies Produced Using Jar Choriocarcinoma Cells

Utilizing the above described procedures, monoclonal antibodies which react immunologically with cytotrophoblasts are produced using JAR choriocarcinoma cells as the immunogen. In the procedure, rats are immunized, and the immunized spleen cells are fused with SP2/0 cells. The resulting hybridomas are screened using the above described procedures. Two clones have been isolated which react immunologically only with cytotrophoblasts. One of these clones is named B4F2.

The JAR human choriocarcinoma line is produced utilizing the procedure of Patillo and Gey (1968), and is grown in DME H16 containing 10% FCS, 1% glutamine, and 50 micrograms/ml gentamicin.

### Detection of First Trimester Cytotrophoblastsin Maternal Blood

Cytotrophoblast cells, isolated as described above, are added to samples of peripheral blood to determine whether these cells can be identified by the anticytotrophoblast monoclonal antibodies. Cytotrophoblasts are purified from abortuses of male fetuses, and added to samples of peripheral blood, at concentrations ranging from 1/10 to 1/10,000. The monoclonal antibodies are incubated with the blood samples under conditions which allow immunological reactivity. The antibody containing samples are then subjected to high speed cell sorting. The resulting separated antibody-cell complexes are deposited on glass slides, and are fixed and stained using fluorescence hybridization with Y-specific probes to confirm their fetal origin.

Alternatively, the Matrigel culture system can be used to enrich for fetal cells in maternal blood. Blood depleted of macrophages will be plated on Matrigel-coated filters and the invasive cytotrophoblast cells collected on the opposite side of the filter. We will also use Matrigel-coated coverslips for growing trophoblast cells isolated from maternal blood.

The following material is on deposit under the terms of the Budapest Treaty with the American Type Culture Collection (ATCC), 12301 Parklawn Dr., Rockville, Maryland 20852, and has been assigned the following Accession Number.

| | | |
|---|---|---|
| | **ATCC No.** | **Deposit Date** |
| Rat Hybridoma, P1B5 (67) | HB 10097 | April 4, 1989 |

The deposited material mentioned herein is intended for convenience only, and is not required to practice the present invention in view of the description herein.

### COMMERCIAL UTILITY

The various embodiments of the invention have a variety of commercial utilities. The monoclonal antibodies produced by the hybridomas isolated by the screening procedures described herein are, because of their specificity, useful for the detection and isolation of circulating cytotrophoblasts in maternal blood. This in turn, provides methods of prenatal diagnosis which are relatively noninvasive, and which may be performed during the first trimester of pregnancy, or early in the second trimester of pregnancy. In addition the monoclonal antibodies are useful in a method for the detection of preeclampsia, prior to the development of overt symptoms, which is based upon an increase in the concentration of cytotrophoblasts in the maternal blood. The screening procedures for hybridomas described herein are useful for isolating hybridomas which produce monoclonal antibodies which are specific for cytotrophoblasts, which in turn, have the above described uses.

### References Cited Herein

Albini et al. (1987) Cancer Res. 47:3239-3245.
Attwood and Park (1960), J. Obstet. Gynec. Brit. Comm. 68:611.
Beer et al. (1972), J. Exp. Med. 135:1177.
Brunette and Till (1971), J. Memb. Biol. 15:215.
Butterworth and Loke (1985), J. Cell Sci. 76:189.
Covone et al. (1984), Lancet, Oct. 13, p. 841-842.
Douglas et al. (1959), Am. J. Obstet. Gynec. 78:960.
Enders (1968), Am. J. Anat. 122:419.
Erkell and Schirrmacher (1988) Cancer Res. 48:6933-6937.
Fisher et al. (1985), J. Cell. Biochem. 27:31.
Fisher, S.J., Cui, T.-Y., Zhang, L., Hartman, L., Grahl, K., Guo-Yang, Z., Tarpey, J. and Damiky, C. (in press) Adhesive and Degradative Properties of Human Placental Cytotrophoblast Cells In Vitro. J. Cell Biology.
Fisher, S.J., Sutherland, A., Moss, L., Hartman, L., Crowley, E., Bernfield, M., Calarco, P. and Damsky, C. (in press) Adhesive Interactions of Murine and Human Trophoblast Cells. Trophoblast Research.
Gilbert and Migeon (1975), Cell 5:11.
Gospodarowicz et al. (1983), J. Cell. Physiol. 114:191.
Gospodarowicz et al. (1984), Proc. Natl. Acad. Sci. 81:6963.
Herzenberg et al. (1979), Proc. Natl. Acad. Sci. 76:1453.
Kawata et al. (1984), J. Exp. Med. 160:633.
Khong et al. (1986), Cell Tissue Res. 246:189.
Kennett et al. (1980) in Monoclonal Antibodies, pp. 365-367 (Plenum Press, N.Y.).
Kliman et al. (1986), Endocrinology 118:1567.
Knudsen (1985), Anal. Biochem. 147:285.
Kramer et al. (1982), J. Biol. Chem. 257:2078.
Kramer et al. (1983), Int. J. Cancer 26:639.
Kramer et al. (1984), J. Natl. Cancer Inst. 72:889.
McIntyre and Faulk (1979), Proc. Natl. Acad. Sci. 76:4029.
Newman et al. (1983), Histochem. J. 15:543.
Parks and Herzenberg (1982) in Methods in Cell Biology, vol. 10, pp. 277-295 (Academic Press, N.Y.).
Patillo and Gey (1968), Cancer Res. 28:1231.
Pijnenborg et al. (1981), Placenta 2:71.
Siebers et al. (1975), Humangenetik 28:273.
Zillacus et al. (1975), Scan. J. Haematol. 15:333.

## Claims (Claims for the following Contracting State(s): AT, BE, CH, DE, DK, FR, GB, IT, LI, LU, NL, SE)

1. A method for preparing a hybridoma which produces monoclonal antibodies which are:
(i) reactive specifically with the cytotrophoblast layer in sections of first trimester human placental chorionic villi;
(ii) reactive *in vitro* with cytotrophoblast cells of the invasive type; and
(iii) not reactive with other cells in the peripheral blood;
the method comprising:
a. providing a purified preparation of first trimester cytotrophoblast cells from chorionic villi from which the layer of syncytiotrophoblastic cells has been removed;
b. immunizing an individual with the preparation of step a;
c. immortalizing antibody-producing cells of the individual immunized in step b; and
d. isolating clones of antibody-producing immortal cells which produce antibodies that have properties (i) to (iii).

2. The method according to claim 1, wherein the monoclonal antibodies are also:
(iv) reactive with purified preparations of dissociated cytotrophoblast cells of both the precursor and invasive types.

3. A hybridoma or progeny thereof, obtainable by the method according to claim 1 or 2, which secretes monoclonal antibodies reactive with first trimester cytotrophoblast cells and not reactive with peripheral blood cells.

4. A monoclonal antibody, obtainable from the hybridoma according to claim 3, which is reactive with first trimester invasive cytotrophoblast cells and not reactive with peripheral blood cells.

5. A method for isolating first trimester foetal cytotrophoblast cells from maternal blood comprising:
a. providing a sample of maternal blood;
b. interacting the maternal blood sample with monoclonal antibodies obtainable from the hybridoma according to claim 3, which are reactive with first trimester invasive cytotrophoblast cells and not reactive with peripheral blood cells, under conditions which allow an immunologically specific reaction; and
c. isolating the antibody-cytotrophoblast complexes from the remainder of the maternal blood components.

6. The method of claim 5, further comprising enriching the maternal blood sample for cytotrophoblasts by interacting the sample with monoclonal antibodies which bind to epitopes on cytotrophoblasts and other cells but do not bind to blood cells, and isolating antibody-cell complexes, wherein the enriching precedes step b. of claim 5.

7. The method of claim 6, wherein the monoclonal antibodies are anticytokeratins.

8. The method of claim 5, further comprising enriching the maternal blood sample for cytotrophoblasts by interacting the sample with antibodies which bind to cells in the peripheral blood which are not cytotrophoblasts, and removing the antibody-cell complexes, wherein the enriching precedes step b. of claim 5.

9. The method of claim 8, wherein the antibodies bind monocytes.

10. The method cf claim 5, wherein the isolating of the complexes is performed in a fluorescence-activated cell sorter.

11. The method of claim 5, wherein the antibodies are attached to a solid phase.

12. The method of claim 5, wherein the antibodies are attached to magnetic beads, and wherein the isolating is accomplished with a magnetic separating device.

13. The method of claim 8, wherein the antibodies are attached to a solid phase.

14. The method of claim 13, wherein the antibodies are attached to magnetic beads, and wherein the isolating is accomplished with a magnetic separating device.

15. A method for detecting first trimester fetal cytotrophoblasts in maternal blood comprising:
a. providing a sample maternal blood;
b. interacting the maternal blood sample with monoclonal antibodies obtainable from the hybridoma according to claim 3, which are reactive with first trimester invasive cytotrophoblast cells and not reactive with peripheral blood cells, under conditions which allow an immunologically specific reaction; and
c. detecting the formation of antibody-cell complexes.

16. The method of claim 15, wherein the detecting is by a device which allows image analysis.

17. The method of claim 16, wherein the detecting is by a device which monitors the flow of a suspension suspected of containing antibody-cell complexes.

18. The hybridoma ATCC No. 10097 and progeny thereof.

## Claims (Claims for the following Contracting State(s): ES, GR)

1. A method for preparing a hybridoma which produces monoclonal antibodies which are:
(i) reactive specifically with the cytotrophoblast layer in sections of first trimester human placental chorionic villi;
(ii) reactive *in vitro* with cytotrophoblast cells of the invasive type; and
(iii) not reactive with other cells in the peripheral blood;
the method comprising:
a. providing a purified preparation of first trimester cytotrophoblast cells from chorionic villi from which the layer of syncytiotrophoblastic cells has been removed;
b. immunizing an individual with the preparation of step a;
c. immortalizing antibody-producing cells of the individual immunized in step b; and
d. isolating clones of antibody-producing immortal cells which produce antibodies that have properties (i) to (iii).

2. The method according to claim 1, wherein the monoclonal antibodies are also:
(iv) reactive with purified preparations of dissociated cytotrophoblast cells of both the precursor and invasive types.

3. A method for isolating first trimester foetal cytotrophoblast cells from maternal blood comprising:
a. providing a sample of maternal blood;
b. interacting the maternal blood sample with monoclonal antibodies obtainable from a hybridoma obtainable by the method according to claim 1 or 2, which are reactive with first trimester invasive cytotrophoblast cells and not reactive with peripheral blood cells, under conditions which allow an immunologically specific reaction; and
c. isolating the antibody-cytotrophoblast complexes from the remainder of the maternal blood components.

4. The method of claim 3, further comprising enriching the maternal blood sample for cytotrophoblasts by interacting the sample with monoclonal antibodies which bind to epitopes on cytotrophoblasts and other cells but do not bind to blood cells, and isolating antibody-cell complexes, wherein the enriching precedes step b. of claim 3.

5. The method of claim 4, wherein the monoclonal antibodies are anticytokeratins.

6. The method of claim 3, further comprising enriching the maternal blood sample for cytotrophoblasts by interacting the sample with antibodies which bind to cells in the peripheral blood which are not cytotrophoblasts, and removing the antibody-cell complexes, wherein the enriching precedes step b. of claim 3.

7. The method of claim 6, wherein the antibodies bind monocytes.

8. The method of claim 3, wherein the isolating of the complexes is performed in a fluorescence-activated cell sorter.

9. The method of claim 3, wherein the antibodies are attached to a solid phase.

10. The method of claim 3, wherein the antibodies are attached to magnetic beads, and wherein the isolating is accomplished with a magnetic separating device.

11. The method of claim 6, wherein the antibodies are attached to a solid phase.

12. The method of claim 11, wherein the antibodies are attached to magnetic beads, and wherein the isolating is accomplished with a magnetic separating device.

13. A method for detecting first trimester fetal cytotrophoblasts in maternal blood comprising:
a. providing a sample maternal blood;
b. interacting the maternal blood sample with monoclonal antibodies obtainable from a hybridoma obtainable by the method according to claim 1 or 2, which are reactive with first trimester invasive cytotrophoblast cells and not reactive with peripheral blood cells, under conditions which allow an immunologically specific reaction; and
c. detecting the formation of antibody-cell complexes.

14. The method of claim 13, wherein the detecting is by a device which allows image analysis.

15. The method of claim 14, wherein the detecting is by a device which monitors the flow of a suspension suspected of containing antibody-cell complexes.

16. A hybridoma or progeny thereof, obtainable by the method according to claim 1 or 2, which secretes monoclonal antibodies reactive with first trimester cytotrophoblast cells and not reactive with peripheral blood cells.

17. A monoclonal antibody, obtainable from the hybridoma according to claim 16, which is reactive with first trimester invasive cytotrophoblast cells and not reactive with peripheral blood cells.

18. The hybridoma ATCC No. 10097 and progeny thereof.

## Patentansprüche (Patentansprüche für folgende(n) Vertragsstaat(en): AT, BE, CH, DE, DK, FR, GB, IT, LI, LU, NL, SE)

1. Verfahren zur Herstellung eines Hybridoms, das monoklonale Antikörper produziert, die:
(i) spezifisch mit der Zytotrophoblastschicht in Schnitten von humanen plazentaren Chorionzotten des ersten Trimesters reagieren,
(ii) *in vitro* mit Zytotrophoblastzellen vom invasiven Typ reagieren und
(iii) mit anderen Zellen im peripheren Blut nicht reagieren,
wobei das Verfahren umfaßt:
a) eine aufgereinigte Präparation von Zytotrophoblastzellen des ersten Trimesters aus Chorionzotten, aus denen die Schicht der Synzytiotrophoblastzellen entfernt worden ist, bereitzustellen,
b) ein Individuum mit der Präparation aus Stufe a) zu immunisieren,
c) Antikörper-produzierende Zellen des in Stufe b) immunisierten Individuums unsterblich zu machen und
d) Klone von unsterblichen, Antikörper-produzierenden Zellen zu isolieren, die Antikörper produzieren, die die Eigenschaften (i) bis (iii) aufweisen.

2. Verfahren nach Anspruch 1, wobei die monoklonalen Antikörper gleichfalls:
(iv) mit aufgereinigten Präparationen von dissoziierten Zytotrophoblastzellen sowohl vom Vorläufertyp als auch vom invasiven Typ reagieren.

3. Hybridom oder Nachkomme desselben, erhältlich durch das Verfahren nach Anspruch 1 oder 2, das bzw. der monoklonale Antikörper sezerniert, die mit Zytotrophoblastzellen des ersten Trimesters reagieren und mit Zellen des peripheren Bluts nicht reagieren.

4. Monoklonaler Antikörper, erhältlich aus dem Hybridom nach Anspruch 3, der mit invasiven Zytotrophoblastzellen des ersten Trimesters reagiert und mit Zellen des peripheren Bluts nicht reagiert.

5. Verfahren zur Isolierung fötaler Zytotrophoblastzellen des ersten Trimesters aus mütterlichem Blut, umfassend:
a) eine Probe von mütterlichem Blut bereitzustellen,
b) die mütterliche Blutprobe mit monoklonalen Antikörpern, die aus dem Hybridom nach Anspruch 3 erhalten werden können, die mit invasiven Zytotrophoblastzellen des ersten Trimesters reagieren und mit Zellen des peripheren Bluts nicht reagieren, unter Bedingungen, die eine immunologisch spezifische Reaktion erlauben, wechselwirken zu lassen und
c) die Antikörper-Zytotrophoblast-Komplexe aus den übrigen Bestandteilen des mütterlichen Bluts zu isolieren.

6. Verfahren nach Anspruch 5, umfassend ferner, die mütterliche Blutprobe hinsichtlich Zytotrophoblasten anzureichern, indem man die Probe mit monoklonalen Antikörpern, die an Epitope auf Zytotrophoblasten und anderen Zellen binden, aber nicht an Blutzellen binden, wechselwirken läßt und Antikörper-Zell-Komplexe isoliert, wobei die Anreicherung der Stufe b) des Anspruchs 5 vorangeht.

7. Verfahren nach Anspruch 6, wobei die monoklonalen Antikörper anti-Zytokeratine sind.

8. Verfahren nach Anspruch 5, umfassend ferner, die mütterliche Blutprobe hinsichtlich Zytotrophoblasten anzureichern, indem man die Probe mit Antikörpern, die an Zellen in dem peripheren Blut, die keine Zytotrophoblasten sind, binden, wechselwirken läßt und die Antikörper-Zell-Komplexe entfernt, wobei die Anreicherung der Stufe b) des Anspruchs 5 vorangeht.

9. Verfahren nach Anspruch 8, wobei die Antikörper Monozyten binden.

10. Verfahren nach Anspruch 5, wobei die Isolierung der Komplexe in einer Fluoreszenzaktivierten Zellsortiervorrichtung ausgeführt wird.

11. Verfahren nach Anspruch 5, wobei die Antikörper an eine feste Phase angeheftet sind.

12. Verfahren nach Anspruch 5, wobei die Antikörper an magnetische Kömchen (beads) angeheftet sind und wobei die Isolierung mit einer magnetischen Abtrennvorrichtung bewerkstelligt wird.

13. Verfahren nach Anspruch 8, wobei die Antikörper an eine feste Phase angeheftet sind.

14. Verfahren nach Anspruch 13, wobei die Antikörper an magnetische Körnchen (beads) angeheftet sind und wobei die Isolierung mit einer magnetischen Abtrennvorrichtung bewerkstelligt wird.

15. Verfahren zum Nachweisen von fötalen Zytotrophoblasten des ersten Trimesters in mütterlichem Blut, umfassend:
a) eine Probe von mütterlichem Blut bereitzustellen,
b) die mütterliche Blutprobe mit monoklonalen Antikörpern, die aus dem Hybridom nach Anspruch 3 erhalten werden können, die mit invasiven Zytotrophoblastzellen des ersten Trimesters reagieren und mit Zellen des peripheren Bluts nicht reagieren, unter Bedingungen, die eine immunologisch spezifische Reaktion erlauben, wechselwirken zu lassen und
c) die Bildung von Antikörper-Zell-Komplexen nachzuweisen.

16. Verfahren nach Anspruch 15, wobei der Nachweis durch eine Vorrichtung erfolgt, die eine Bildanalyse ermöglicht.

17. Verfahren nach Anspruch 16, wobei der Nachweis durch eine Vorrichtung erfolgt, die den Fluß einer Suspension überwacht, von der vermutet wird, daß sie Antikörper-Zell-Komplexe enthält.

18. Hybridom ATCC Nr. 10097 und Nachkommen davon.

## Patentansprüche (Patentansprüche für folgende(n) Vertragsstaat(en): ES, GR)

1. Verfahren zur Herstellung eines Hybridoms, das monoklonale Antikörper produziert, die:
(i) spezifisch mit der Zytotrophoblastschicht in Schnitten von humanen plazentaren Chorionzotten des ersten Trimesters reagieren,
(ii) *in vitro* mit Zytotrophoblastzellen vom invasiven Typ reagieren und
(iii) mit anderen Zellen im peripheren Blut nicht reagieren,
wobei das Verfahren umfaßt:
a) eine aufgereinigte Präparation von Zytotrophoblastzellen des ersten Trimesters aus Chorionzotten, aus denen die Schicht der Synzytiotrophoblastzellen entfernt worden ist, bereitzustellen,
b) ein Individuum mit der Präparation aus Stufe a) zu immunisieren,
c) Antikörper-produzierende Zellen des in Stufe b) immunisierten Individuums unsterblich zu machen und
d) Klone von unsterblichen, Antikörper-produzierenden Zellen zu isolieren, die Antikörper produzieren, die die Eigenschaften (i) bis (iii) aufweisen.

2. Verfahren nach Anspruch 1, wobei die monoklonalen Antikörper gleichfalls:
(iv) mit aufgereinigten Präparationen von dissoziierten Zytotrophoblastzellen sowohl vom Vorläufertyp als auch vom invasiven Typ reagieren.

3. Verfahren zur Isolierung fötaler Zytotrophoblastzellen des ersten Trimesters aus mütterlichem Blut, umfassend:
a) eine Probe von mütterlichem Blut bereitzustellen,
b) die mütterliche Blutprobe mit monoklonalen Antikörpern, die aus einem durch das Verfahren nach Anspruch 1 oder 2 erhältlichen Hybridom erhalten werden können, die mit invasiven Zytotrophoblastzellen des ersten Trimesters reagieren und mit Zellen des peripheren Bluts nicht reagieren, unter Bedingungen, die eine immunologisch spezifische Reaktion erlauben, wechselwirken zu lassen und
c) die Antikörper-Zytotrophoblast-Komplexe aus den übrigen Bestandteilen des mütterlichen Bluts zu isolieren.

4. Verfahren nach Anspruch 3, umfassend ferner, die mütterliche Blutprobe hinsichtlich Zytotrophoblasten anzureichern, indem man die Probe mit monoklonalen Antikörpern, die an Epitope auf Zytotrophoblasten und anderen Zellen binden, aber nicht an Blutzellen binden, wechselwirken läßt und Antikörper-Zell-Komplexe isoliert, wobei die Anreicherung der Stufe b) des Anspruchs 3 vorangeht.

5. Verfahren nach Anspruch 4, wobei die monoklonalen Antikörper anti-Zytokeratine sind.

6. Verfahren nach Anspruch 3, umfassend ferner, die mütterliche Blutprobe hinsichtlich Zytotrophoblasten anzureichern, indem man die Probe mit Antikörpern, die an Zellen in dem peripheren Blut, die keine Zytotrophoblasten sind, binden, wechselwirken läßt und die Antikörper-Zell-Komplexe entfernt, wobei die Anreicherung der Stufe b) des Anspruchs 3 vorangeht.

7. Verfahren nach Anspruch 6, wobei die Antikörper Monozyten binden.

8. Verfahren nach Anspruch 3, wobei die Isolierung der Komplexe in einer Fluoreszenzaktivierten Zellsortiervorrichtung ausgeführt wird.

9. Verfahren nach Anspruch 3, wobei die Antikörper an eine feste Phase angeheftet sind.

10. Verfahren nach Anspruch 3, wobei die Antikörper an magnetische Körnchen (beads) angeheftet sind und wobei die Isolierung mit einer magnetischen Abtrennvorrichtung bewerkstelligt wird.

11. Verfahren nach Anspruch 6, wobei die Antikörper an eine feste Phase angeheftet sind.

12. Verfahren nach Anspruch 11, wobei die Antikörper an magnetische Körnchen (beads) angeheftet sind und wobei die Isolierung mit einer magnetischen Abtrennvorrichtung bewerkstelligt wird.

13. Verfahren zum Nachweisen von fötalen Zytotrophoblasten des ersten Trimesters in mütterlichem Blut, umfassend:
a) eine Probe von mütterlichem Blut bereitzustellen,
b) die mütterliche Blutprobe mit monoklonalen Antikörpern, die aus einem durch das Verfahren nach Anspruch 1 oder 2 erhältlichen Hybridom erhalten werden können, die mit invasiven Zytotrophoblastzellen des ersten Trimesters reagieren und mit Zellen des peripheren Bluts nicht reagieren, unter Bedingungen, die eine immunologisch spezifische Reaktion erlauben, wechselwirken zu lassen und
c) die Bildung von Antikörper-Zell-Komplexen nachzuweisen.

14. Verfahren nach Anspruch 13, wobei der Nachweis durch eine Vorrichtung erfolgt, die eine Bildanalyse ermöglicht.

15. Verfahren nach Anspruch 14, wobei der Nachweis durch eine Vorrichtung erfolgt, die den Fluß einer Suspension überwacht, von der vermutet wird, daß sie Antikörper-Zell-Komplexe enthält.

16. Hybridom oder Nachkomme desselben, erhältlich durch das Verfahren nach Anspruch 1 oder 2, das bzw. der monoklonale Antikörper sezerniert, die mit Zytotrophoblastzellen des ersten Trimesters reagieren und mit Zellen des peripheren Bluts nicht reagieren.

17. Monoklonaler Antikörper, erhältlich aus dem Hybridom nach Anspruch 16, der mit invasiven Zytotrophoblastzellen des ersten Trimesters reagiert und mit Zellen des peripheren Bluts nicht reagiert.

18. Hybridom ATCC Nr. 10097 und Nachkommen davon.

## Revendications (Revendications pour l'(les) Etat(s) contractant(s) suivant(s): AT, BE, CH, DE, DK, FR, GB, IT, LI, LU, NL, SE)

1. Procédé pour préparer un hybridome produisant des anticorps monoclonaux qui
(i) ont une réactivité spécifique vis-à-vis de la couche de cytotrophoblastes dans des sections de villosités choriales de placenta humain du premier trimestre,
(ii) ont une réactivité *in vitro* vis-à-vis de cytotrophoblastes de type invasif, et
(iii) ne sont pas réactifs vis-à-vis d'autres cellules du sang périphérique,
lequel procédé comprend les étapes consistant
a. à prendre une préparation purifiée de cytotrophoblastes du premier trimestre provenant des villosités choriales et dont on a éliminé la couche de cellules syncytiotrophoblastiques,
b. à immuniser un individu avec la préparation de l'étape a,
c. à immortaliser les cellules productrices d'anticorps de l'individu immunisé dans l'étape b, et
d. à isoler les clones de cellules immortelles produisant des anticorps qui ont les propriétés (i) à (iii).

2. Procédé conforme à la revendication 1 dans lequel les anticorps monoclonaux sont également
(iv) réactifs vis-à-vis de préparations purifiées de cytotrophoblastes dissociés de type précurseur et de type invasif.

3. Hybridome ou descendance de celui-ci, que l'on peut obtenir par le procédé conforme à la revendication 1 ou 2, sécrétant des anticorps monoclonaux réactifs vis-à-vis de cytotrophoblastes du premier trimestre et non réactifs vis-à-vis de cellules du sang périphérique.

4. Anticorps monoclonaux, que l'on peut obtenir à partir de l'hybridome conforme à la revendication 3, lesquels anticorps sont réactifs vis-à-vis de cytotrophoblastes invasifs du premier trimestre et ne sont pas réactifs vis-à-vis de cellules du sang périphérique.

5. Procédé pour isoler des cytotrophoblastes foetaux du premier trimestre à partir du sang maternel comprenant les étapes consistant
a. à prélever un échantillon de sang maternel,
b. à mettre en interaction l'échantillon de sang maternel avec des anticorps monoclonaux que l'on peut obtenir à partir de l'hybridome conforme à la revendication 3, lesquels anticorps sont réactifs vis-à-vis de cytotrophoblastes invasifs du premier trimestre et ne sont pas réactifs vis-à-vis de cellules du sang périphérique, dans des conditions qui permettent une réaction immunologique spécifique, et
c. à isoler les complexes anticorps-cytotrophoblastes du reste des composants du sang maternel.

6. Procédé conforme à la revendication 5 comprenant en outre l'enrichissement de l'échantillon de sang maternel en cytotrophoblastes par interaction de l'échantillon avec des anticorps monoclonaux qui se lient à des épitopes de cytotrophoblastes et d'autres cellules mais qui ne se lient pas à des cellules sanguins, et par isolement des complexes anticorps-cellules, cet enrichissement précédant l'étape b de la revendication 5.

7. Procédé conforme à la revendication 6 dans lequel les anticorps monoclonaux sont des anticytokératines.

8. Procédé conforme à la revendication 5 comprenant en outre l'enrichissement de l'échantillon de sang maternel en cytotrophoblastes par interaction de l'échantillon avec des anticorps qui se lient à des cellules du sang périphérique autres que cytotrophoblastes, et par élimination des complexes anticorps-cellules, cet étape d'enrichissement précédant l'étape b de la revendication 5.

9. Procédé conforme à la revendication 8 dans lequel les anticorps se lient à des monocytes.

10. Procédé conforme à la revendication 5 dans lequel l'isolement des complexes se fait au moyen d'un trieur de cellules activées par fluorescence.

11. Procédé conforme à la revendication 5 dans lequel les anticorps sont immobilisés sur une phase solide.

12. Procédé conforme à la revendication 5 dans lequel les anticorps sont immobilisés sur des billes magnétiques et dans lequel l'isolement se fait au moyen d'un dispositif de séparation magnétique.

13. Procédé conforme à la revendication 8 dans lequel les anticorps sont immobilisés sur une phase solide.

14. Procédé conforme à la revendication 13 dans lequel les anticorps sont immobilisés sur des billes magnétiques et dans lequel l'isolement se fait au moyen d'un dispositif de séparation magnétique.

15. Procédé de détection de cytotrophoblastes foetaux de premier trimestre dans du sang maternel, comprenant les étapes consistant
a. à prélever un échantillon de sang maternel,
b. à faire interagir l'échantillon de sang maternel avec des anticorps monoclonaux que l'on peut obtenir à partir de l'hybridome conforme à la revendication 3 et qui sont réactifs vis-à-vis de cytotrophoblastes invasifs du premier trimestre et non réactifs vis-à-vis de cellules du sang périphérique, dans des conditions qui permettent une réaction immunologique spécifique, et
c. à détecter la formation de complexes anticorps-cellules.

16. Procédé conforme à la revendication 15 dans lequel la détection se fait par un dispositif d'analyse d'image.

17. Procédé conforme à la revendication 16 dans lequel la détection se fait par un dispositif de surveillance de l'écoulement d'une suspension de cellules suspectée de contenir des complexes anticorps-cellules.

18. Hybridome ATCC n° 10097 et sa descendance.

## Revendications (Revendications pour l'(les) Etat(s) contractant(s) suivant(s): ES, GR)

1. Procédé pour préparer un hybridome produisant des anticorps monoclonaux qui
(i) ont une réactivité spécifique vis-à-vis de la couche de cytotrophoblastes dans des sections de villosités choriales de placenta humain du premier trimestre,
(ii) ont une réactivité *in vitro* vis-à-vis de cytotrophoblastes de type invasif, et
(iii) ne sont pas réactifs vis-à-vis d'autres cellules du sang périphérique,
lequel procédé comprend les étapes consistant
a. à prendre une préparation purifiée de cytotrophoblastes du premier trimestre provenant des villosités choriales et dont on a éliminé la couche de cellules syncytiotrophoblastiques,
b. à immuniser un individu avec la préparation de l'étape a,
c. à immortaliser les cellules productrices d'anticorps de l'individu immunisé dans l'étape b, et
d. à isoler les clones de cellules immortelles produisant des anticorps qui ont les propriétés (i) à (iii).

2. Procédé conforme à la revendication 1 dans lequel les anticorps monoclonaux sont également
(iv) réactifs vis-à-vis de préparations purifiées de cytotrophoblastes dissociés de type précurseur et de type invasif.

3. Procédé pour isoler des cytotrophoblastes foetaux du premier trimestre à partir du sang maternel comprenant les étapes consistant
a. à prélever un échantillon de sang maternel,
b. à mettre en interaction l'échantillon de sang maternel avec des anticorps monoclonaux que l'on peut obtenir à partir de l'hybridome que l'on peut obtenir grâce au procédé de la revendication 1 ou 2, lesquels anticorps sont réactifs vis-à-vis de cytotrophoblastes invasifs du premier trimestre et ne sont pas réactifs vis-à-vis de cellules du sang périphérique, dans des conditions qui permettent une réaction immunologique spécifique, et
c. à isoler les complexes anticorps-cytotrophoblastes du reste des composants du sang maternel.

4. Procédé conforme à la revendication 3 comprenant en outre l'enrichissement de l'échantillon de sang maternel en cytotrophoblastes par interaction de l'échantillon avec des anticorps monoclonaux qui se lient à des épitopes de cytotrophoblastes et d'autres cellules mais qui ne se lient pas à des cellules sanguins, et par isolement des complexes anticorps-cellules, cet enrichissement précédant l'étape b de la revendication 3.

5. Procédé conforme à la revendication 4 dans lequel les anticorps monoclonaux sont des anticytokératines.

6. Procédé conforme à la revendication 3 comprenant en outre l'enrichissement de l'échantillon de sang maternel en cytotrophoblastes par interaction de l'échantillon avec des anticorps qui se lient à des cellules du sang périphérique autres que cytotrophoblastes, et par élimination des complexes anticorps-cellules, cet étape d'enrichissement précédant l'étape b de la revendication 3.

7. Procédé conforme à la revendication 6 dans lequel les anticorps se lient à des monocytes.

8. Procédé conforme à la revendication 3 dans lequel l'isolement des complexes se fait au moyen d'un trieur de cellules activées par fluorescence.

9. Procédé conforme à la revendication 3 dans lequel les anticorps sont immobilisés sur une phase solide.

10. Procédé conforme à la revendication 3 dans lequel les anticorps sont immobilisés sur des billes magnétiques et dans lequel l'isolement se fait au moyen d'un dispositif de séparation magnétique.

11. Procédé conforme à la revendication 6 dans lequel les anticorps sont immobilisés sur une phase solide.

12. Procédé conforme à la revendication 11 dans lequel les anticorps sont immobilisés sur des billes magnétiques et dans lequel l'isolement se fait au moyen d'un dispositif de séparation magnétique.

13. Procédé de détection de cytotrophoblastes foetaux du premier trimestre dans du sang maternel, comprenant les étapes consistant
a. à prélever un échantillon de sang maternel,
b. à faire interagir l'échantillon de sang maternel avec des anticorps monoclonaux que l'on peut obtenir à partir de l'hybridome que l'on peut obtenir grâce au procédé de la revendication 1 ou 2 et qui sont réactifs vis-à-vis de cytotrophoblastes invasifs du premier trimestre et non réactifs vis-à-vis de cellules du sang périphérique, dans des conditions qui permettent une réaction immunologique spécifique, et
c. à détecter la formation de complexes anticorps-cellules.

14. Procédé conforme à la revendication 13 dans lequel la détection se fait par un dispositif d'analyse d'image.

15. Procédé conforme à la revendication 14 dans lequel la détection se fait par un dispositif de surveillance de l'écoulement d'une suspension de cellules suspectée de contenir des complexes anticorps-cellules.

16. Hybridome ou descendance de celui-ci, que l'on peut obtenir par le procédé conforme à la revendication 1 ou 2, sécrétant des anticorps monoclonaux réactifs vis-à-vis de cytotrophoblastes du premier trimestre et non réactifs vis-à-vis de cellules du sang périphérique.

17. Anticorps monoclonaux, que l'on peut obtenir à partir de l'hybridome conforme à la revendication 16, lesquels anticorps sont réactifs vis-à-vis de cytotrophoblastes invasifs du premier trimestre et ne sont pas réactifs vis-à-vis de cellules du sang périphérique.

18. Hybridome ATCC n° 10097 et sa descendance.
